(19) Europäisches Patentamt
European Patent Office
Office européen des brevets

(11) Veröffentlichungsnummer: **0 138 183**
**A2**

(12) # EUROPÄISCHE PATENTANMELDUNG

(21) Anmeldenummer: **84112038.9**

(22) Anmeldetag: **08.10.84**

(51) Int. Cl.⁴: **C 07 C 43/29**, C 07 F 9/40, C 07 D 213/64, A 01 N 31/14, A 01 N 43/40

(30) Priorität: **18.10.83 CH 5648/83**
**17.08.84 CH 3946/84**

(43) Veröffentlichungstag der Anmeldung: **24.04.85**
**Patentblatt 85/17**

(84) Benannte Vertragsstaaten: **AT BE CH DE FR GB IT LI NL**

(71) Anmelder: **F. HOFFMANN-LA ROCHE & CO.**
**Aktiengesellschaft, CH-4002 Basel (CH)**

(72) Erfinder: **Winternitz, Paul, Dr., Am Pfisterhölzli 50,**
**CH-8606 Greifensee (CH)**
Erfinder: **Zurflüh, René, Dr., Lohzelgstrasse 13,**
**CH-8122 Pfaffhausen (CH)**

(74) Vertreter: **Lederer, Franz, Dr. et al, Patentanwälte Dr.**
**Lederer Franz Meyer-Roxlau Reiner F.**
**Lucile-Grahn-Strasse 22, D-8000 München 80 (DE)**

(54) **Diaryläther und deren Verwendung als Unkrautbekämpfungsmittel.**

(57) Die Erfindung betrifft neue Diaryläther der Formel

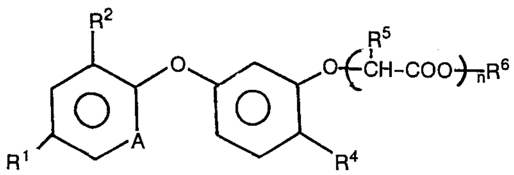

I

worin A, $R^1$, $R^2$, $R^4$, $R^5$, n und $R^6$ die in der Beschreibung angegebenen Bedeutungen besitzen, Verfahren zu deren Herstellung, Unkrautbekämpfungsmittel, die diese Verbindungen als Wirkstoffe enthalten, sowie die Verwendung solcher Verbindungen bzw. Mittel zur Unkrautbekämpfung.

0138183

F.Hoffmann-La Roche & Co. Aktiengesellschaft,Basel,Schweiz

8. Okt. 1984

RAN 6102/45

# Diaryläther und deren Verwendung als Unkrautbekämpfungsmittel

Die Erfindung betrifft Diaryläther der allgemeinen Formel

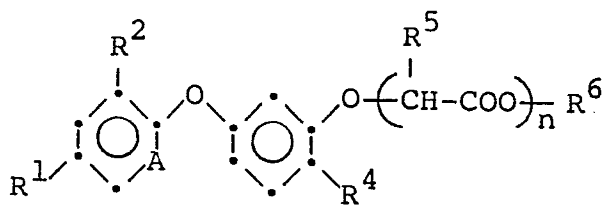

I

worin A $CR^3$ oder N,

$R^1$ Halogen oder Trifluormethyl,

$R^2$ und $R^3$ unabhängig voneinander Wasserstoff, Halogen, Nitro oder Cyano,

$R^4$ Halogen, Nitro oder Cyano,

$R^5$ Wasserstoff oder $C_{1-3}$-Alkyl,

n 0 oder 1,

$R^6$ eine der folgenden Gruppen (a) - (c)

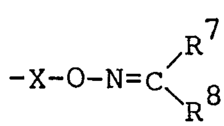

(a)

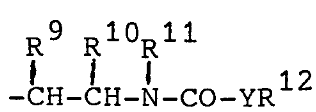

(b)

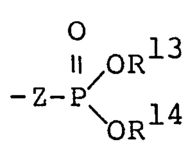

(c)

$R^7$ $C_{1-6}$-Alkyl,
$R^8$ $C_{1-6}$-Alkyl oder $C_{1-6}$-Alkoxy,
oder $R^7$ und $R^8$ zusammen mit dem Kohlenstoffatom, an das sie geknüpft sind, einen $C_{5-7}$-Cycloalkanring,
X Methylen, Aethylen oder Aethyliden,
$R^9$ und $R^{10}$ unabhängig voneinander Wasserstoff oder $C_{1-4}$-Alkyl,
$R^{11}$ Wasserstoff oder Methyl,
$R^{12}$ $C_{1-4}$-Alkyl oder 2-Chloräthyl,
Y Sauerstoff oder Schwefel,
$R^{13}$ und $R^{14}$ unabhängig voneinander $C_{1-6}$-Alkyl
und Z $C_{1-3}$-Alkylen bedeuten.

Die Verbindungen der Formel I besitzen herbizide Eigenschaften und eignen sich als Wirkstoffe von Unkrautbekämpfungsmitteln. Somit umfasst die Erfindung auch Unkrautbekämpfungsmittel, welche Verbindungen der Formel I als Wirkstoffe enthalten, Verfahren zur Herstellung dieser Verbindungen sowie die Verwendung solcher Verbindungen bzw. Mittel zur Bekämpfung von Unkräutern.

In der obigen Formel I umfasst "Halogen" Fluor, Chlor, Brom und Jod. Unter "$C_{1-3}$-Alkyl", "$C_{1-4}$-Alkyl" bzw. "$C_{1-6}$-Alkyl" sind sowohl geradkettige als auch verzweigte Alkylgruppen zu verstehen, wie Methyl, Aethyl, n-Propyl, Isopropyl, n-Butyl, Isobutyl, tert.Butyl, n-Pentyl, Isoamyl, neo-Pentyl und n-Hexyl. Dies gilt auch für den Alkylteil der $C_{1-6}$-Alkoxygruppe. Auch $C_{1-3}$-Alkylen kann geradkettig oder verzweigt sein; es ist also Methylen, Aethylen, Aethyliden, 1- oder 2-Methyläthylen, Propyliden oder Trimethylen.

Das Vorhandensein mindestens eines asymmetrischen Kohlenstoffatoms in den Verbindungen der Formel I hat zur Folge, dass die Verbindungen in isomeren Formen auftreten können, welche in enantiomerer oder diastereomerer Beziehung zueinander stehen. Das gleiche gilt für die Verbindungen der Formel I, in der zusätzlich $R^{13}$ und $R^{14}$ unterschiedliche Bedeutung besitzen. Die isomeren Formen weisen optische Aktivität auf. Durch das Vorliegen der Stickstoff-Kohlenstoff-Doppelbindung in den Verbindungen der Formel I, worin $R^6$ eine Gruppe (a) bedeutet, tritt für jene Verbindungen, worin $R^7$ und $R^8$ unterschiedliche Bedeutung haben, zusätzlich geometrische Isomerie auf. Unabhängig davon kommt auch in gewissen Fällen eine Atropisomerie vor. Die Formel I soll demnach all diese möglichen isomeren Formen sowie die Racemate umfassen.

Eine interessante Untergruppe von Verbindungen der Formel I besteht aus denjenigen Verbindungen der Formel I, worin A CH, $R^1$ Trifluormethyl, $R^2$ Halogen, $R^4$ Halogen oder Nitro, $R^7$ und $R^8$ unabhängig voneinander $C_{1-6}$-Alkyl, $R^{12}$ $C_{1-4}$-Alkyl und Y Sauerstoff bedeuten.

Unabhängig voneinander bedeuten A vorzugsweise CH; $R^1$ vorzugsweise Trifluormethyl; $R^2$ vorzugsweise Chlor; $R^4$ vorzugsweise Nitro; $R^5$ vorzugsweise Wasserstoff oder Methyl, insbesondere Methyl; und $R^7$ und $R^8$ vorzugsweise Methyl oder Aethyl.

Besonders bevorzugte Verbindungen der Formel I sind:

2-[5-(o-Chlor-p-trifluormethyl-phenoxy)-2-nitro-phenoxy]-propionsäure-isopropylidenaminooxymethylester,
2-[5-(o-Chlor-p-trifluormethyl-phenoxy)-2-nitro-phenoxy]-propionsäure-(2-isopropylidenaminooxy-äthyl)ester und
N-{2-/2-[5-(o-Chlor-p-trifluormethyl-phenoxy)-2-nitrophenoxy]-propionyloxy/-äthyl}-N-methyl-carbaminsäure-methylester.

Weitere bevorzugte Verbindungen der Formel I sind:

2-[5-(o-Chlor-p-trifluormethyl-phenoxy)-2-nitro-phenoxy]-äthylcarbaminsäure-äthylester,
2-/2-[5-(o-Chlor-p-trifluormethyl-phenoxy)-2-nitro-phenoxy]-propionyloxy/-äthylcarbaminsäure-äthylester und
/2-[5-(o-Chlor-p-trifluormethyl-phenoxy)-2-nitro-phenoxy]-propionyloxy/methylphosphonsäure-diäthylester.

Das erfindungsgemässe Verfahren zur Herstellung der Verbindungen der Formel I ist dadurch gekennzeichnet, dass man

a) ein Phenol der allgemeinen Formel

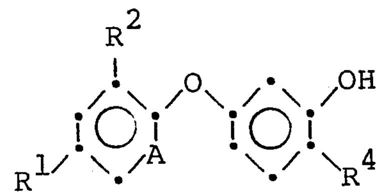

II

worin A, $R^1$, $R^2$ und $R^4$ die oben angegebenen Bedeutungen besitzen,

oder ein Alkalimetallsalz davon, mit einer Verbindung der allgemeinen Formel

$$Q-\left(\overset{R^5}{\overset{|}{C}}H-COO\right)_n-R^6 \qquad III$$

worin $R^5$, n und $R^6$ die oben angegebenen Bedeutungen besitzen

und Q eine Abgangsgruppe bedeutet,

umsetzt,

b) eine Säure der allgemeinen Formel

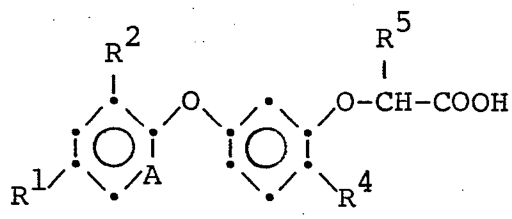

IV

worin A, $R^1$, $R^2$, $R^4$ und $R^5$ die oben angegebenen Bedeutungen besitzen,

oder ein reaktionsfähiges Derivat davon, mit einer Verbindung der allgemeinen Formel

$$U-R^6 \qquad V$$

worin $R^6$ die oben angegebene Bedeutung besitzt

und U Hydroxy oder eine Abgangsgruppe bedeutet,

umsetzt,

c) eine Verbindung der allgemeinen Formel

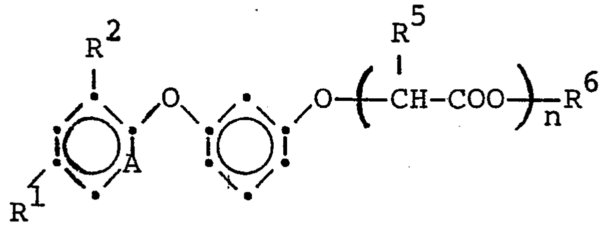

VI

worin A, $R^1$, $R^2$, $R^5$, n und $R^6$ die oben angegebenen Bedeutungen besitzen,

mit einem Metallnitrat behandelt oder

d) ein o-Dinitrobenzolderivat der allgemeinen Formel

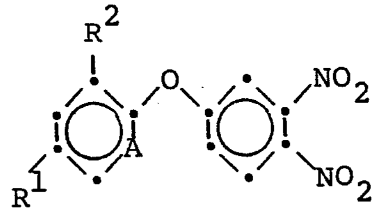

VII

worin A, $R^1$ und $R^2$ die oben angegebenen Bedeutungen besitzen,

mit einem Alkohol der allgemeinen Formel

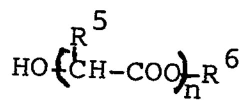

VIII

worin $R^5$, n und $R^6$ die oben angegebenen Bedeutungen besitzen,

oder einem Alkalimetallsalz davon umsetzt.

Bei der Verfahrensvariante a) steht der Ausdruck "Alkalimetallsalz" insbesondere für das Natrium-, Kalium- oder Lithiumsalz des diesbezüglichen Phenols der Formel II. Der Ausdruck "Abgangsgruppe" für Q in der Formel III steht vorzugsweise für Chlor, Brom, Jod, Mesyloxy oder Tosyloxy, oder für eine Gruppe $-N^{\oplus}R^{15}R^{16}R^{17}$, worin $R^{15}$, $R^{16}$ und $R^{17}$ unabhängig voneinander Wasserstoff oder nieder Alkyl bedeuten oder zusammen mit dem Stickstoffatom einen Ring, wie Pyridinium, bilden, wobei als geeignetes Anion insbesondere Halogenid, z.B. $Cl^{\ominus}$ oder $Br^{\ominus}$, in Frage kommt. Im Falle der Verwendung eines Ausgangsmaterials der Formel III, in der Q eine Gruppe $-N^{\oplus}R^{15}R^{16}R^{17}$ ist, wird das freie Phenol der Formel II eingesetzt.

Die Umsetzung nach Verfahrensvariante a) kann zweckmässigerweise durchgeführt werden, indem man ein Alkalimetallsalz des Phenols der Formel II mit einem Halogenid - insbesondere dem Chlorid, Bromid oder Jodid - , dem Mesylat oder dem Tosylat der Formel III in einem inerten organischen Lösungsmittel, insbesondere einem dipolaren aprotischen Lösungsmittel, wie Dimethylformamid oder Dimethylsulfoxid, umsetzt. Die Reaktionstemperatur ist nicht kritisch, und man arbeitet im allgemeinen bei Temperaturen zwischen 0°C und 120°, vorzugsweise zwischen 20°C und 70°C. Als Vorstufe wird das Phenol der Formel II in das Alkalimetallsalz übergeführt, und zwar vorzugsweise unter Verwendung von Natriumhydrid oder Kaliumhydroxid unter azeotroper Wasserentfernung.

Die Umsetzung nach Verfahrensvariante a) kann zweckmässigerweise auch dadurch durchgeführt werden, dass man das freie Phenol der Formel II mit einem Halogenid - insbesondere dem Chlorid, Bromid oder Jodid -, dem Mesylat oder dem Tosylat der Formel III in Gegenwart einer geeigneten anorganischen Base, z.B. eines Alkalimetall- oder Erdalkalimetallcarbonats oder -bicarbonats, oder einer organischen Base, wie eines tert.Amins, z.B. Triäthylamin, Dimethylanilin, Pyridin oder, insbesondere, 1,8-Diaza-bicyclo-[4,5,0]undec-7-en, umsetzt. Zudem erfolgt die Umsetzung zweckmässigerweise in einem polaren aprotischen Lösungsmittel, wie einem aliphatischen Keton, z.B. Aceton oder 2-Butanon, Dimethylformamid oder Acetonitril, und gegebenenfalls in Gegenwart einer katalytischen Menge Natriumjodid, Kaliumjodid oder eines quaternären Ammoniumjodids, z.B. Tetrabutylammoniumjodid, bei Temperaturen zwischen 0°C und 150°C, vorzugsweise zwischen 20°C und 100°C.

Bedeutet bei der Verfahrensvariante a) Q in der Formel III eine Gruppe $-N^{\oplus}R^{15}R^{16}R^{17}$, wie diese oben näher beschrieben ist, so wird die Umsetzung vorzugsweise in einem Hydroxylgruppen enthaltenden Medium, z.B. in Gegenwart von Natrium-oder Kaliumhydroxid, durchgeführt. Es erfolgt

die Umsetzung mit dem freien Phenol der Formel II zweckmässigerweise in einem inerten organischen Verdünnungsmittel, wie einem aromatischen Kohlenwasserstoff, z.B. Toluol, einem aliphatischen oder cyclischen Aether, z.B. Dimethoxyäthan oder Dioxan, einem aprotischen dipolaren Lösungsmittel, z.B. Dimethylformamid oder Acetonitril, oder einem Gemisch solcher Verdünnungsmittel, bei Temperaturen zwischen 70°C und 160°C, vorzugsweise zwischen 100°C und 120°.

Bei der Verfahrensvariante b), die zu denjenigen Verbindungen der Formel I führt, in der n 1 bedeutet, handelt es sich um eine Veresterung, die nach an sich bekannten Methoden durchgeführt werden kann. Somit bedeutet der Ausdruck "reaktionsfähiges Derivat" insbesondere ein Halogenid, das Imidazolid oder das Anhydrid der diesbezüglichen Säure der Formel IV. Bevorzugte Abgangsgruppen U in der Formel V sind Chlor, Brom, Jod, Mesyloxy und Tosyloxy, ferner eine Gruppe $-N^{\oplus}R^{15}R^{16}R^{17}$, wie diese im Zusammenhang mit der Verfahrensvariante a) näher beschrieben ist.

Die Veresterung einer freien Säure der Formel IV mit einer Verbindung der Formel V, in der U eine Abgangsgruppe, wie Chlor, Brom, Jod, Mesyloxy oder Tosyloxy, bedeutet, wird zweckmässigerweise in Gegenwart einer Base bzw. eines Säureacceptors durchgeführt. Man kann zu diesem Zwecke alle üblicherweise verwendbaren anorganischen und organischen säurebindenden Mittel benützen, vorzugsweise aber Alkalimetall- und Erdalkalimetallcarbonate und -bicarbonate, und tertiäre Amine, z.B. Triäthylamin, Dimethylanilin, 1,8-Diaza-bicyclo-[4,5,0]undec-7-en und Pyridin. Die Umsetzung erfolgt zweckmässigerweise in einem inerten Verdünnungsmittel, vorzugsweise einem inerten organischen Lösungsmittel, wie einem aliphatischen oder cyclischen Aether, z.B. Diäthyläther oder Tetrahydrofuran, einem Kohlenwasserstoff, z.B. n-Hexan, Benzol oder Toluol, einem aliphatischen Keton, z.B. Aceton oder 2-Butanon, einem chlorierten Kohlenwasserstoff, z.B. Dichlormethan, Chloroform oder Tetrachlorkohlenstoff, oder einem dipolaren aprotischen Lösungsmittel,

z.B. Dimethylformamid, Dimethylsulfoxid, N-Methylpyrrolidon, Sulfolan oder Hexamethylphosphorsäuretriamid, bei Temperaturen zwischen Raumtemperatur und dem Rückflusstemperatur des Reaktionsgemisches.

Bei der Veresterung einer freien Säure der Formel IV mit einer Verbindung der Formel V, in der U als Abgangsgruppe eine Gruppe $-N^{\oplus}R^{15}R^{16}R^{17}$ bedeutet, wird vorzugsweise in einem Hydroxylgruppen enthaltenden Medium, z.B. in Gegenwart von Natrium- oder Kaliumhydroxid, umgesetzt. Die Umsetzung wird zweckmässigerweise in einem inerten organischen Verdünnungsmittel, wie einem aromatischen Kohlenwasserstoff, z.B. Toluol, einem aliphatischen oder cyclischen Aether, z.B. Dimethoxyäthan oder Dioxan, einem aprotischen dipolaren Lösungsmittel, z.B. Dimethylformamid oder Acetonitril, oder einem Gemisch solcher Verdünnungsmittel, bei Temperaturen zwischen 70°C und 160°C, vorzugsweise zwischen 100°C und 120°C, durchgeführt.

Die Umsetzung einer freien Säure der Formel IV mit einer Verbindung der Formel V, in der U Hydroxy bedeutet, wird zweckmässigerweise in einem inerten organischen Verdünnungsmittel, wie einem aliphatischen oder cyclischen Aether, z.B. Diäthyläther, Diisopropyläther, 1,2-Dimethoxyäthan, Tetrahydrofuran oder Dioxan, einem chlorierten Kohlenwasserstoff, z.B. Dichlormethan, Chloroform, Tetrachlorkohlenstoff oder Trichloräthan, oder einem aromatischen Kohlenwasserstoff, z.B. Benzol, Toluol oder einem Xylol, und bei Temperaturen zwischen 0°C und der Rückflusstemperatur des Reaktionsgemisches durchgeführt. Zudem erfolgt die Umsetzung zweckmässigerweise in Gegenwart eines sauren Katalysators bzw. eines kondensierenden Mittels, wie Schwefelsäure, Salzsäure, p-Toluolsulfonsäure, Dicyclohexylcarbodiimid oder Carbonyldiimidazol. Vorzugsweise wird Carbonyldiimidazol als kondensierendes Mittel benützt, wobei bevorzugt in Gegenwart eines Aethers als Verdünnungsmittel umgesetzt wird. Es wird die Säure der Formel IV, z.B. in einem inerten organischen Verdünnungsmittel,

insbesondere einem aliphatischen oder cyclischen Aether, gelöst oder suspendiert und die Lösung bzw. Suspension zu einer Lösung oder Suspension von Carbonyldiimidazol in dem gleichen Verdünnungsmittel gegeben. Nach Beendigung der resultierenden Entwicklung von Kohlendioxid wird das Gemisch mit einer Lösung des Alkohols der Formel V, die zuvor mit einer katalytischen Menge Natrium oder Natriumhydrid bis zur Beendigung von Wasserstoffentwicklung versetzt wurde, versetzt. Die Umsetzung erfolgt vorzugsweise in einem Temperaturbereich zwischen Raumtemperatur und ca. 50°C ; sie ist normalerweise innert ca. 2 Stunden beendet.

Im Falle der Umsetzung eines reaktionsfähigen Derivats einer Säure der Formel IV mit einer Verbindung der Formel V steht U in letzterer Formel für Hydroxy. Die Umsetzung wird zweckmässigerweise in einem inerten Verdünnungsmittel, wie einem aliphatischen oder cyclischen Aether, z.B. Diäthyläther oder Tetrahydrofuran, einem Kohlenwasserstoff, z.B. n-Hexan, Benzol oder Toluol, oder einem chlorierten Kohlenwasserstoff, z.B. Dichlormethan, Chloroform oder Tetrachlorkohlenstoff, und bei Raumtemperatur oder erhöhter Temperatur, z.B. bis zur Rückflusstemperatur des Reaktionsgemisches, durchgeführt.

Bei Verwendung eines Säurehalogenides der Säure der Formel IV wird die Umsetzung zweckmässigerweise in Gegenwart eines säurebindenden Mittels und vorzugsweise bei Temperaturen zwischen 0°C und 40°C, insbesondere zwischen 0°C und 10°C, durchgeführt. Als geeignete säurebindende Mittel eignen sich anorganische Basen, z.B. Alkalimetall- und Erdalkalimetallcarbonate und -bicarbonate, sowie organische Basen, z.B. tertiäre Amine, insbesondere Triäthylamin oder Pyridin. Das bevorzugte Säurehalogenid ist das Säurechlorid. Bei Verwendung des Anhydrids der Säure der Formel IV erfolgt die Umsetzung zweckmässigerweise in Gegenwart eines säurebindenden Mittels, insbesondere einer organischen Base, wie eines tertiären Amins, z.B. Triäthylamin, Dimethylanilin oder Pyridin, bei Temperaturen zwischen 0°C

und 40°C, insbesondere bei Raumtemperatur, oder ohne säurebindendes Mittel bei erhöhter Temperatur.

Bei der Verfahrensvariante c), die zu denjenigen Verbindungen der Formel I führt, in der $R^4$ Nitro bedeutet, steht der Ausdruck "Metallnitrat" insbesondere für Kupfer(II)-, Eisen(III)-, Cobalt(II)-, Cobalt(III)-, Nickel(III)-, Chrom(III)-, Mangan(II)- oder Aluminium(III)-nitrat. Die bevorzugten Metallnitrate sind Kupfer(II)-, Eisen(III)- und Aluminium(III)-nitrat. Die Umsetzung wird zweckmässigerweise in Gegenwart von Essigsäureanhydrid und eines inerten Verdünnungsmittels, insbesondere eines organischen Lösungsmittels, und bei Temperaturen zwischen 0°C und 100°C, insbesondere zwischen 10°C und 50°C, durchgeführt. Als Verdünnungsmittel dient vorzugsweise Essigsäureanhydrid. Es wird vorzugsweise mit einem Ueberschuss an Metallnitrat gearbeitet, und zwar im allgemeinen mit einem 1- bis 10-molaren Ueberschuss, vorzugsweise einem 1,0- bis 1,5-molaren Ueberschuss, an Metallnitrat. Solche Verfahren sind z.B. von J.B. Menke in Rec. Trav. Chim. Pays-Bas <u>44</u>, 141 und 269 (1925), von G. Bacharach in J. Amer. Chem. Soc. <u>49</u>, 1522 (1927) und von A.M. Talati und B. Shah in Ind. J. Chem. <u>11</u>, 1076 (1973) beschrieben.

Die Verfahrensvariante d) führt ebenfalls zu denjenigen Verbindungen der Formel I, in der $R^4$ Nitro bedeutet. Unter dem Ausdruck "Alkalimetallsalz" des Alkohols der Formel VIII ist insbesondere das Natrium-, Kalium- oder Lithiumsalz zu verstehen. Die Umsetzung erfolgt zweckmässigerweise in einem inerten Verdünnungsmittel, insbesondere einem organischen Lösungsmittel, wie Tetrahydrofuran oder Dioxan, und in Gegenwart einer Base, wie eines Alkalimetallhydroxids, z.B. Natrium- oder Kaliumhydroxid. Man arbeitet im allgemeinen in einem Temperaturbereich zwischen Raum- und Rückflusstemperatur, vorzugsweise zwischen 40°C und 100°C. Ein solches Verfahren ist z.B. in der Deutschen Offenlegungsschrift Nr. 2.926.829 beschrieben.

Das erhaltene Produkt kann nach an sich bekannten Methoden isoliert und gereinigt werden.

Sofern keine gezielte Synthese zur Isolierung reiner Isomerer durchgeführt wird, fällt das Produkt normalerweise als Gemisch zweier oder mehrerer Isomerer an. Die Isomeren können nach an sich bekannten Methoden aufgetrennt werden. Gewünschtenfalls können sie auch durch Synthese aus entsprechenden optisch aktiven Ausgangsmaterialien hergestellt werden.

Die Ausgangsmaterialien der Formeln II und IV sowie deren Alkalimetallsalze bzw. reaktionsfähige Derivate sind entweder bekannt oder können in an sich bekannter Weise hergestellt werden. Die als Ausgangsmaterialien der Verfahrensvariante a) verwendeten Verbindungen der Formel III, die auch die in der Verfahrensvariante b) verwendeten Ausgangsmaterialien der Formel V, in der U eine Abgangsgruppe bedeutet, umfassen, lassen sich in an sich bekannter Weise herstellen, z.B. gemäss den nachfolgenden Reaktionsschema:

Reaktionsschemata

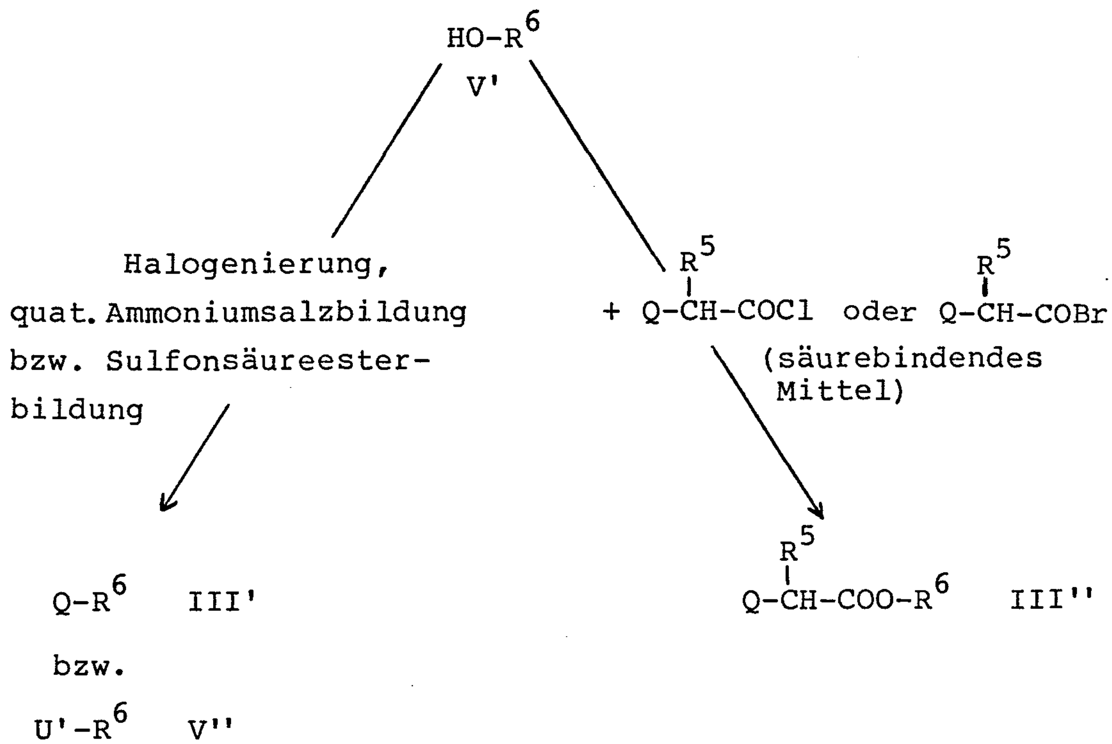

In den obigen Reaktionsschemata besitzen $R^5$, $R^6$ und Q die oben angegebenen Bedeutungen; U' bedeutet eine Abgangsgruppe, insbesondere Chlor, Brom, Jod, Mesyloxy oder Tosyloxy, oder eine Gruppe $-N^{\oplus}R^{15}R^{16}R^{17}$, wie diese im Zusammenhang mit der Verfahrensvariante a) näher beschrieben ist. Die Reaktionsbedingungen sind dem Fachmann geläufig.

Die restlichen Ausgangsmaterialien der Formel V, d.h. diejenigen Verbindungen der Formel V, in der U Hydroxy bedeutet, und die obigen Verbindungen der Formel V', sind entweder bekannt oder können in an sich bekannter Weise hergestellt werden.

Die Ausgangsmaterialien der Formel VI können dadurch hergestellt werden, dass man ein Phenol der allgemeinen Formel

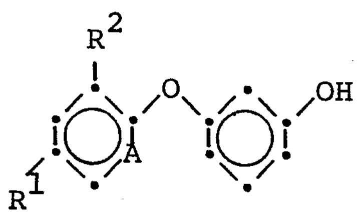

IX

worin A, $R^1$ und $R^2$ die oben angegebenen Bedeutungen besitzen,

oder ein Alkalimetallsalz davon, wie das Natrium-, Kalium- oder Lithiumsalz, mit einer Verbindung der oben angegebenen allgemeinen Formel III umsetzt. Die Umsetzung erfolgt zweckmässigerweise unter denjenigen Reaktionsbedingungen, die oben im Zusammenhang mit der Verfahrensvariante a) beschrieben sind.

Die als Ausgangsmaterialien der Verfahrensvariante d) verwendeten o-Dinitrobenzolderivate der Formel VII können analog dem in der Deutschen Offenlegungsschrift Nr. 2.926.829 beschriebenen Verfahren zur Herstellung von 1-(2-Chlor-4-trifluormethyl-phenoxy)-3,4-dinitrobenzol hergestellt werden.

Die als Ausgangsmaterialien der Verfahrensvariante d)

verwendeten Alkohole der Formel VIII und deren Alkalimetallsalze lassen sich in an sich bekannter Weise herstellen, z.B. durch Umsetzung eines Alkohols der oben angegebenen allgemeinen Formel V' mit einem Säurechlorid der Formel

$$HO-\overset{\overset{R^5}{|}}{C}H-COCl$$

worin $R^5$ die oben angegebene Bedeutung besitzt, in Gegenwart eines säurebindenden Mittels, und gegebenenfalls anschliessende Alkalimetallsalzbildung.

Die Verbindungen der Formel I besitzen herbizide Eigenschaften und eignen sich besonders zur Bekämpfung von Unkräutern, insbesondere von monokotylen Ungräsern, wie Mohrenhirse (Sorghum bicolor), Hühnerhirse (Echinochloa crus-galli) und Fingerhirse (Digitaria spp.), sowie dikotylen Ungräsern, wie Chinesischem Hanf (Abutilon theophrasti), Weissem Gänsefuss (Chenopodium album), Rauhaarigem Amarant (Amaranthus retroflexus), Ackersenf (Sinapis arvensis), Stechapfel (Datura stramonium), Prukwinde (Ipomoea spp.) und Spitzklette (Xanthium pensylvanicum) in diversen Nutzpflanzenkulturen, insbesondere in Reis- und Getreidekulturen.

Im allgemeinen genügt eine Konzentration von 0,01 bis 3 kg Wirkstoff der Formel I/ha, vorzugsweise 0,01 bis 1 kg Wirkstoff der Formel I/ha, um den gewünschten herbiziden Effekt zu erzielen.

Die Verbindungen der Formel I sind sowohl Vorauflauf-Herbizide als auch Nachauflauf-Herbizide.

Das erfindungsgemässe Unkrautbekämpfungsmittel ist dadurch gekennzeichnet, dass es eine wirksame Menge mindestens einer Verbindung der Formel I, wie oben definiert,

sowie Formulierungshilfsstoffe enthält. Das Mittel enthält zweckmässigerweise zumindest einen der folgenden Formulierungshilfsstoffe: feste Trägerstoffe; Lösungs- bzw. Dispersionsmittel; Tenside (Netz- und Emulgiermittel); Dispergatoren (ohne Tensidwirkung); und Stabilisatoren. Unter Verwendung solcher und anderer Hilfsstoffe können die Verbindungen der Formel I, also die herbiziden Wirkstoffe, in die üblichen Formulierungen übergeführt werden, wie Stäube, Pulver, Granulate, Lösungen, Emulsionen, Suspensionen, emulgierbare Konzentrate, Pasten und dergleichen.

Die Verbindungen der Formel I sind im allgemeinen wasserunlöslich und können nach den für wasserunlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln und/oder von Dispergatoren, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chlor-

äthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen sein, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäuren, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel z.B. Epichlorhydrin, Phenylglycidäther und Soyaepoxide; Antioxidantien z.B. Gallussäureester und Butylhydroxytoluol; UV-Absorber, z.B. substituierte Benzophenone, Diphenylacrylonitrilsäureester und Zimtsäureester; und Deaktivatoren, z.B. Salze der Aethylendiaminotetraessigsäure und Polyglykole.

Die erfindungsgemässen Unkrautbekämpfungsmittel können zusätzlich zu den Verbindungen der Formel I Synergisten und andere Wirkstoffe, z.B. Insektizide, Akarizide, Bakterizide, anderweitige Herbizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,001 und 95 Gewichtsprozent, vorzugsweise zwischen 5 und 75 Gewichtsprozent einer bzw. mehrerer Verbindungen der Formel I als Wirkstoff(e). Sie können z.B. in einer Form vorliegen, die sich für die

Lagerung und den Transport eignet. In solchen Formulierungen, z.B. emulgierbaren Konzentraten, ist die Wirkstoffkonzentration normalerweise im höheren Bereich, vorzugsweise zwischen 10 und 75 Gewichtsprozent, insbesondere zwischen 25 und 50 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,1 bis 10 Gewichtsprozent, insbesondere ca. 1 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine Verbindung der Formel I, mit festem Trägerstoff vermischt werden, z.B. durch Zusammenmahlen; oder man kann den festen Trägerstoff mit einer Lösung oder Suspension des Wirkstoffes imprägnieren und dann das Lösungs- bzw. Dispersionsmittel durch Abdunsten, Erhitzen oder Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindung der Formel I kann auch mit einem Tensid und einem festen Trägerstoff zur Bildung eines netzbaren Pulvers vermischt werden, welches in Wasser dispergierbar ist, oder sie kann mit einem festen vorgranulierten Trägerstoff zur Bildung eines granulatförmigen Produktes vermischt werden.

Wenn gewünscht, kann die Verbindung der Formel I in einem mit Wasser nicht mischbaren Lösungsmittel, wie beispielsweise einem hochsiedenden Kohlenwasserstoff, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer erfindungsgemässen Verbindung bzw. einem erfindungsgemässen Unkrautbekämpfungsmittel behandelt.

Die nachfolgenden Beispiele dienen zur näheren Erläuterung der Erfindung.

I. Herstellung der Wirkstoffe der Formel I:

Beispiel 1

Zu einer Lösung von 3,0 g Diäthyl-methyl-isopropylidenaminooxymethyl-ammoniumjodid in 10 ml Methanol werden 4,2 g Silberoxid gegeben, und das Gemisch wird 1 Stunde bei Raumtemperatur und im Dunkeln gerührt. Man filtriert die festen Anteile ab und gibt zum Filtrat 3,0 g 5-(o-Chlor-p-trifluormethyl-phenoxy)-2-nitrophenol sowie 50 ml Toluol zu. Nach azeotropem Abdestillieren des Methanols und des bei der Reaktion gebildeten Wassers wird das Gemisch 15 Minuten auf Rückflusstemperatur erhitzt, abgekühlt und auf 100 ml Wasser gegossen. Die organische Phase wird abgetrennt und die wässrige Phase mit 50 ml Aethylacetat extrahiert. Anschliessend werden die vereinigten organischen Phasen der Reihe nach mit 100 ml 2N Salzsäure, mit 100 ml 5%iger Natriumbicarbonatlösung und zweimal mit jeweils 100 ml Wasser gewaschen. Das Lösungsmittel wird abgedampft und der Rückstand an der zehnfachen Menge Kieselgel unter Verwendung von Diäthyläther/n-Hexan (1:9) als Laufmittel chromatographisch gereinigt. Man erhält das 2-Propanon-O-/[5-(o-chlor-p-trifluormethyl-phenoxy)-2-nitrophenoxy]-methyl/oxim als farbloses Oel (2,9 g), $n_D^{20}$ 1,5443; Massenspektrum m/e 418; $^1$H-NMR ($CDCl_3$; 60 MHz) 7,90 ppm (d, 1H), 7,80 ppm (d, 1H), 7,65 ppm (dd, 1H), 7,25 ppm (d, 1H), 6,65 ppm (dd, 1H), 5,70 ppm (s, 2H), 1,78 ppm (d, 6H).

Beispiel 2

Eine Lösung von 1,65 g Diäthyl-methyl-isopropylidenaminooxymethyl-ammoniumjodid in 10 ml Methanol wird in Gegenwart von 2,3 g Silberoxid 1 Stunde bei Raumtemperatur und im Dunkeln gerührt. Man filtriert die festen Anteile ab, gibt zum Filtrat 2,0 g 2-[5-(o-Chlor-p-trifluormethyl-phenoxy)-2-nitrophenoxy]-propionsäure sowie 50 ml Toluol zu und destilliert das Methanol und das gebildete Wasser azeotrop ab. Danach wird das Gemisch eine halbe Stunde auf

Rückflusstemperatur erhitzt, abgekühlt und auf 100 ml Wasser gegossen. Man trennt die organische Phase ab und extrahiert die wässrige Phase zweimal mit jeweils 50 ml Aethylacetat. Anschliessend werden die vereinigten organischen Phasen der Reihe nach mit 50 ml 2N Salzsäure, mit 50 ml 5%iger Natriumbicarbonatlösung und zweimal mit jeweils 50 ml Wasser gewaschen. Das Lösungsmittel wird abgedampft und der Rückstand an der zehnfachen Menge Kieselgel unter Verwendung von n-Hexan/Aethylacetat (1:1) als Laufmittel chromatographisch gereinigt. Man erhält den 2-[5-(o-Chlor-p-trifluormethyl-phenoxy)-2-nitrophenoxy]-propionsäure-isopropylidenaminooxymethylester, $n_D^{20}$ 1,5346; Massenspektrum m/e 490; $^1$H-NMR ($CDCl_3$; 60 MHz) 7,90 ppm (d, 1H), 7,80 ppm (d, 1H), 7,60 ppm (dd, 1H), 7,20 ppm (d, 1H), 6,60 ppm (d, 1H), 6,55 ppm (dd, 1H), 5,75 ppm (s, 2H), 4,75 ppm (q, 1H), 1,90 ppm (d, 6H), 1,72 ppm (d, 6H).

## Beispiel 3

Eine Lösung von 4,0 g 5-(o-Chlor-p-trifluormethyl-phenoxy)-1-nitrophenol in 30 ml Acetonitril wird mit 2,0 g Kaliumcarbonat während 1 Stunde auf Rückflusstemperatur erhitzt. Nach Zugabe katalytischer Mengen Kaliumjodid und Tetrabutylammoniumjodid tropft man eine Lösung von 1,9 g 2-Chlor-äthylcarbaminsäure-methylester in 15 ml Acetonitril in das warme Gemisch und hält das Reaktionsgemisch während 35 Stunden bei Rückflusstemperatur. Das abgekühlte Reaktionsgemisch wird filtriert und das Filtrat mit Diäthyläther verdünnt. Die Aetherlösung wird mit 2N Salzsäure, Wasser und gesättigter Kochsalzlösung gewaschen, über wasserfreiem Natriumsulfat getrocknet und unter vermindertem Druck eingedampft. Der ölige Rückstand wird an Kieselgel mit n-Hexan/Aethylacetat (4:1) chromatographisch gereinigt. Man erhält den 2-[5-(o-Chlor-p-trifluormethyl-phenoxy)-2-nitrophenoxy]-äthylcarbaminsäure-methylester, Smp. 91-92°C.

In analoger Weise erhält man beim Einsatz von 2-Chlor-äthylcarbaminsäure-äthylester den 2-[5-(o-Chlor-p-trifluormethyl-phenoxy)-2-nitrophenoxy]-äthylcarbaminsäure-äthylester, Smp. 70-71°C.

## Beispiel 4

2,0 g 5-(o-Chlor-p-trifluormethyl-phenoxy)-2-nitrophenol und 1,0 g Kaliumcarbonat werden in 30 ml Acetonitril während 15 Minuten auf Rückflusstemperatur erhitzt. Dann tropft man eine Lösung von 1,68 g 2-(2-Brompropionyloxy)-äthylcarbaminsäure-methylester in 10 ml Acetonitril während 5 Minuten zu und lässt während ca. 16 Stunden bei Rückflusstemperatur reagieren. Zur Aufarbeitung wird auf Eiswasser gegossen und mit Diäthyläther zweimal extrahiert. Die vereinigten Aetherlösungen werden mit 2N Salzsäure, Wasser und gesättigter Kochsalzlösung gewaschen, über wasserfreiem Natriumsulfat getrocknet und eingedampft. Der ölige Rückstand wird durch Chromatographie an Kieselgel mit n-Hexan/Aethylacetat (4:1) gereinigt. Man erhält den 2-/2-[5-(o-Chlor-p-trifluormethyl-phenoxy)-2-nitrophenoxy]-propionyloxy/-äthylcarbaminsäure-methylester, $n_D^{20}$ 1,5258.

In analoger Weise erhält man beim Einsatz von:

- 2-(2-Brompropionyloxy)-äthylcarbaminsäure-äthylester den 2-/2-[5-(o-Chlor-p-trifluormethyl-phenoxy)-2-nitrophenoxy]-propionyloxy/-äthylcarbaminsäure-äthylester, $n_D^{24}$ 1,5185.

- 2-Bromacetyloxy-äthylcarbaminsäure-methylester den 2-/[5-(o-Chlor-p-trifluormethyl-phenoxy)-2-nitrophenoxy]-acetyloxy/-äthylcarbaminsäure-methylester, Smp. 105-108°C.

- 2-Bromacetyloxy-äthylcarbaminsäure-äthylester den 2-/[5-(o-Chlor-p-trifluormethyl-phenoxy)-2-nitrophenoxy]-acetyloxy/-äthylcarbaminsäure-äthylester, Smp. 94-95°C.

- 2-(2-Brompropionyloxy)-propylcarbaminsäure-methylester

den 2-/2-[5-(o-Chlor-p-trifluormethyl-phenoxy)-2-nitro-phenoxy]-propionyloxy/-propylcarbaminsäure-methylester, $^1$H-NMR ($CDCl_3$, 60 MHz) 8,1 - 6,46 ppm (m, 6H), 5,23 - 4,63 ppm (m, 2H), 4,83 ppm (q, 1H), 3,65 ppm (s, 3H), 3,5 - 3,13 ppm (m, 2H), 1,70 ppm (d, 3H), 1,23 ppm (d, 3H).

- 1-Aethyl-2-(2-brompropionyloxy)-äthylcarbaminsäure-äthylester den 1-Aethyl-2-/2-[5-(o-chlor-p-trifluormethyl-phenoxy)-2-nitrophenoxy]-propionyloxy/-äthylcarbaminsäure-äthylester, $^1$H-NMR ($CDCl_3$, 400 MHz) 7,91 ppm (d, 1H), 7,79 ppm (dd, 1H), 7,61 - 7,56 ppm (m, 1H), 7,20 ppm (dd, 1H), 6,67 ppm (breites s, 1H) und 6,59 ppm (d, 1H), 6,51 ppm (dd, 1H), 4,85 ppm (q, 1H), 4,73 - 4,53 ppm (m, 1H), 3,85 - 3,71 ppm (m, 1H), 1,7 und 1,695 ppm (2d, 3H), 1,56 - 1,3 ppm (m, 2H), 1,29 - 1,18 ppm (m, 3H), 0,93 und 0,90 ppm (2t, 3H).

- 2-(2-Brompropionyloxy)-äthylcarbaminsäure-isobutylester den 2-/2-[5-(o-Chlor-p-trifluormethyl-phenoxy)-2-nitro-phenoxy]-propionyloxy/-äthylcarbaminsäure-isobutylester, $^1$H-NMR ($CDCl_3$, 60 MHz) 8,1 - 6,4 ppm (m, 6H), 5,17 - 4,80 ppm (breites s, 1H), 4,86 ppm (q, 1H), 4,23 ppm (t, 2H), 3,80 ppm (d, 2H), 3,66 - 3,26 ppm (m, 2H), 1,70 ppm (d, 3H), 1,90 ppm (m, 1H), 0,93 ppm (d, 6H).

- N-[2-(2-Brompropionyloxy)-äthyl]-N-methyl-carbaminsäure-methylester den N-{2-/2-[5-(o-Chlor-p-trifluormethyl-phenoxy)-2-nitrophenoxy]-propionyloxy/-äthyl} -N-methyl-carbaminsäure-methylester, $^1$H-NMR ($CDCl_3$, 60 MHz) 8,1 - 6,43 ppm (m, 6H), 4,83 ppm (q, 1H), 4,26 ppm (t, 2H), 3,67 ppm (s, 3H), 3,50 ppm (t, 2H), 2,86 ppm (s, 3H), 1,68 ppm (d, 3H).

In analoger Weise erhält man beim Einsatz von 5-(o-Chlor-p-trifluormethyl-phenoxy)-2-chlorphenol und:

- 2-(2-Brompropionyloxy)-äthylcarbaminsäure-methylester den 2-/2-[5-(o-Chlor-p-trifluormethyl-phenoxy) -2-chlor-phenoxy]-propionyloxy/ -äthylcarbaminsäure-methylester. $^1$H-NMR ($CDCl_3$, 60 MHz) 7,85-6,45 ppm (mehrere Signale, 6H), 5,05 ppm (t, 1H), 4,83 ppm (q, 1H, $\gamma$ = ca. 7-8 Hz), 4,39-3,85 ppm (mehrere Signale, 4H), 3,65 ppm (s, 3H), 1,70 ppm (d, 3H, $\gamma$ = ca. 7-8 Hz).

- 2-(2-Brompropionyloxy)-äthylcarbaminsäure-äthylester den 2-/2-[5-(o-Chlor-p-trifluormethyl-phenoxy) -2-chlor-phenoxy]-propionyloxy/ -äthylcarbaminsäure-äthylester. $^1$H-NMR ($CDCl_3$, 60 MHz) 7,85-6,45 ppm (mehrere Signale, 6H), 5,0 ppm (t, 1H), 4,85 ppm (q, 1H, $\gamma$ = ca. 7-8 Hz), 4,40-3,90 ppm (mehrere Signale, 4H), 3,42 ppm (q, 2H, $\gamma$ = ca- 7-8 Hz), 1,72 ppm (d, 3H, $\gamma$ = ca. 7-8 Hz), 1,25 ppm (t, 3H, $\gamma$ = ca. 7-8 Hz).

- 2-Brompropionsäure-(2-isopropylidenaminooxyäthyl)ester den 2-[5-(o-Chlor-p-trifluormethyl-phenoxy) -2-chlor-phenoxy]-propionsäure -(2-isopropylidenaminooxy-äthyl)-ester. $^1$H-NMR ($CDCl_3$, 60 MHz) 7,85-6,45 ppm (mehrere Signale, 6H), 4,80 ppm (q, 1H, $\gamma$ = ca. 7-8 Hz), 4,30 ppm (m, 4H), 1,80 ppm (s, 3H), 1,78 ppm (s, 3H), 1,70 ppm (d, 3H, $\gamma$ = ca. 7-8 Hz).

## Beispiel 5

1,3 g D-2-[5-(o-Chlor-p-trifluormethyl-phenoxy) -2-nitrophenoxy]-propionsäure werden mit 15 ml Thionylchlorid während 1 Stunde auf Rückflusstemperatur erhitzt. Dann wird das überschüssige Thionylchlorid bei vermindertem Druck

abdestilliert und der Rückstand am Hochvakuum getrocknet. Nach Zugabe von 10 ml Methylenchlorid und 0,27 g Pyridin tropft man 0,47 g 2-Hydroxyäthylcarbaminsäure-äthylester in 10 ml Methylenchlorid bei 0-5°C zu und lässt während ca. 16 Stunden bei Raumtemperatur reagieren. Zur Aufarbeitung wird auf Wasser gegossen und dreimal mit Diäthyläther extrahiert. Die Extrakte werden mit 2N Salzsäure, Wasser und gesättigter Kochsalzlösung gewaschen, über wasserfreiem Natriumsulfat getrocknet und eingedampft. Durch Chromatographie an Kieselgel mit n-Hexan/Aethylacetat (4:1) erhält man den 2-/D-2-[5-(o-Chlor-p-trifluormethyl-phenoxy)-2-nitrophenoxy]-propionyloxy/-äthylcarbaminsäure-äthylester, $n_D^{20}$ 1,5362; $[\alpha]_D^{22}$ -18,85° (c = 1,15% in $CHCl_3$).

In analoger Weise erhält man beim Einsatz von D-2-[5-(o-Chlor-p-trifluormethyl-phenoxy)-2-nitrophenoxy]-propionsäure und 2-Hydroxyäthylcarbaminsäure-methylester den 2-/D-2-[5-(o-Chlor-p-trifluormethyl-phenoxy)-2-nitrophenoxy]-propionyloxy/-äthylcarbaminsäure-methylester, $[\alpha]_D^{20}$ - 15,76° (c = 0,83% in $CHCl_3$); $^1H$-NMR ($CDCl_3$, 60 MHz) 8,1 - 6,45 ppm (m, 6H), 5,25 - 4,7 ppm (breites s, 1H), 4,88 ppm (q, 1H), 4,25 ppm (t, 2H), 3,70 ppm (s, 3H), 3,67 - 3,28 ppm (m, 2H), 1,73 ppm (d, 3H).

## Beispiel 6

Zu einer Lösung von 2,0 g DL-2-[5-(o-Chlor-p-trifluormethyl-phenoxy)-2-nitrophenoxy]-propionsäurechlorid und 0,86 g 2-Hydroxyäthylphosphonsäure-diäthylester in 5 ml Methylenchlorid wird bei Raumtemperatur 0,41 g Pyridin zugetropft. Das Reaktionsgemisch wird 1 Stunde bei Raumtemperatur nachgerührt und anschliessend unter vermindertem Druck zur Trockene eingedampft. Der Rückstand wird in Aethylacetat gelöst und die Lösung mit Wasser gewaschen, über wasserfreiem Natriumsulfat getrocknet und zur Trockene eingedampft. Der Rückstand wird an Kieselgel mit Aethylacetat/n-Hexan (1:1) chromatographisch gereinigt. Man erhält den 2-/DL-2-[5-(o-Chlor-p-trifluormethyl-phenoxy)-2-nitrophenoxy]-propion-

yloxy/-äthylphosphonsäure-diäthylester, $^1$H-NMR ($CDCl_3$; 400 MHz) 7,93 ppm (d, 1H), 7,79 ppm (d, 1H), 7,59 ppm (q, 1H), 7,21 ppm (d, 1H), 6,62 ppm (d, 1H), 6,51 ppm (q, 1H), 4,81 ppm (q, 1H), 4,39 ppm (m, 2H), 4,11 ppm (m, 4H), 2,12 ppm (m, 2H), 1,71 ppm (d, 3H), 1,33 ppm (tt, 6H).

In analoger Weise erhält man beim Einsatz von:

- 5-(o-Chlor-p-trifluormethyl-phenoxy)-2-nitrophenoxy-essigsäurechlorid und 2-Hydroxyäthylphosphonsäure-diäthylester den 2-/[5-(o-Chlor-p-trifluormethyl-phenoxy)-2-nitrophenoxy]acetyloxy/-äthylphosphonsäure-diäthylester, $^1$H-NMR ($CDCl_3$; 400 MHz) 7,96 ppm (d, 1H), 7,80 ppm (d, 1H), 7,59 ppm (q, 1H), 7,21 ppm (d, 1H), 6,71 ppm (d, 1H), 6,52 ppm (q, 1H), 4,78 ppm (s, 2H), 4,45 ppm (m, 2H), 4,12 ppm (m, 4H), 2,17 ppm (m, 2H), 1,33 ppm (tt, 6H).

- 2-[5-(o-Chlor-p-trifluormethyl-phenoxy)-2-nitrophenoxy]-propionsäurechlorid und 1-Hydroxyäthylphosphonsäure-diäthylester den 1-/2-[5-(o-Chlor-p-trifluormethyl-phenoxy)-2-nitrophenoxy]-propionyloxy/-äthylphosphonsäure-diäthylester, $^1$H-NMR ($CDCl_3$, 400 MHz) 7,93 ppm und 7,92 ppm (2d, 1H), 7,79 ppm (d, 1H), 7,61 - 7,55 ppm (m, 1H), 7,23 ppm und 7,19 ppm (2d, 1H), 6,68 ppm und 6,58 ppm (2d, 1H), 6,53 ppm und 6,46 ppm (2q, 1H), 5,31 ppm und 5,29 ppm (2q, 1H), 4,88 und 4,85 ppm (2q, 1H), 4,21 - 4,05 ppm (m, 4H), 1,73 ppm und 1,72 ppm (2d, 3H), 1,45 ppm und 1,42 ppm (2q, 3H), 1,36 - 1,26 ppm (m, 6H).

## Beispiel 7

Eine Lösung von 1,85 g 5-(o-Chlor-p-trifluormethyl-phenoxy)-2-nitrophenol-Kaliumsalz und 1,29 g 3-Diäthylphosphopropylbromid in 25 ml absolutem Dimethylsulfoxid wird während 4 Stunden auf 75-80°C erhitzt. Das Lösungsmittel wird nachher weitgehend unter vermindertem Druck abdestilliert, der Rückstand mit Wasser versetzt und die wässrige Lösung auf einen pH-Wert von ca. 6 gebracht. Anschliessend wird

die wässrige Lösung mit Aethylacetat ausgeschüttelt, und die vereinigten organischen Extrakte werden mit Wasser gewaschen, über wasserfreiem Natriumsulfat getrocknet und zur Trockene eingedampft. Durch Chromatographie an Kieselgel mit n-Hexan/Aethylacetat gefolgt von reinem Aethylacetat als Laufmittel wird der Rückstand gereinigt. Man erhält den 3-[5-(o-Chlor-p-trifluormethyl-phenoxy)-2-nitrophenoxy]-propylphosphonsäure-diäthylester, $^1$H-NMR ($CDCl_3$; 400 MHz) 7,95 ppm (d, 1H), 7,80 ppm (d, 1H), 7,58 ppm (q, 1H), 7,20 ppm (d, 1H), 6,69 ppm (d, 1H), 6,45 ppm (q, 1H), 4,11 ppm (m, 6H), 2,17 ppm (m, 2H), 2,02 ppm (m, 2H), 1,32 ppm (tt, 6H).

In analoger Weise erhält man beim Einsatz von:

- 5-(o-Chlor-p-trifluormethyl-phenoxy)-2-nitrophenol-Kaliumsalz und 2-Brompropionsäure-(2-isopropylidenaminooxy-äthyl)ester den 2-[5-(o-Chlor-p-trifluormethyl-phenoxy)-2-nitrophenoxy]-propionsäure-(2-isopropylidenaminooxy-äthyl)-ester, $^1$H-NMR ($CDCl_3$; 400 MHz) 7,93 ppm (d, 1H), 7,79 ppm (d, 1H), 7,58 ppm (q, 1H), 7,18 ppm (d, 1H), 6,59 ppm (d, 1H), 6,49 ppm (q, 1H), 4,81 ppm (q, 1H), 4,37 ppm (m, 2H), 4,15 ppm (t, 2H), 1,84 ppm (s, 3H), 1,79 ppm (s, 3H), 1,71 ppm (d, 3H).

- 5-(o-Chlor-p-trifluormethyl-phenoxy)-2-nitrophenol-Kaliumsalz und (2-Brompropionyloxy)methylphosphonsäure-diäthylester den /2-[5-(o-Chlor-p-trifluormethyl-phenoxy)-2-nitrophenoxy]-propionyloxy/methylphosphonsäure-diäthylester, $^1$H-NMR ($CDCl_3$; 400 MHz) 7,93 ppm (d, 1H), 7,79 ppm (d, 1H), 7,58 ppm (q, 1H), 7,21 ppm (d, 1H), 6,63 ppm (d, 1H), 6,49 ppm (q, 1H), 4,89 ppm (q, 1H), 4,45 ppm (m, 2H), 4,14 ppm (m, 4H), 1,74 ppm (d, 3H), 1,32 ppm (tt, 6H).

## Beispiel 8

Eine Lösung von 2,0 g 2-[5-(o-Chlor-p-trifluormethyl-phenoxy)-2-nitrophenoxy]-propionsäure in 20 ml Dimethylformamid wird zu 0,22 g einer 55%-igen Dispersion von Natriumhydrid in Oel zugetropft. Anschliessend wird das Gemisch auf 50°C erhitzt und 15 Minuten gerührt. Man fügt danach eine Lösung von 1,3 g 3-Brompropylphosphonsäure-diäthylester in 4 ml Dimethylformamid zu und rührt das Reaktionsgemisch 6 Stunden bei 50°C.

Zur Aufarbeitung destilliert man das Lösungsmittel weitgehend unter vermindertem Druck ab, löst den Rückstand in Aethylacetat, und wäscht die Lösung mit Wasser, trocknet sie über wasserfreiem Natriumsulfat und dampft sie ein. Schliesslich wird das Rohprodukt an Kieselgel mit Aethylacetat/n-Hexan (3:1) chromatographisch gereinigt. Man erhält den 3-[2-[5-(o-Chlor-p-trifluormethyl-phenoxy)-2-nitrophenoxy]-propionyloxy] -propylphosphonsäure-diäthylester, $^1$N-NMR ($CDCl_3$, 400 MHz) 7,93 ppm (d, 1H), 7,79 ppm (d, 1H), 7,60 ppm (q, 1H), 7,23 ppm (d, 1H), 6,57 ppm (d, 1H), 6,50 ppm (q, 1H), 4,81 ppm (q, 1H), 4,28-4,00 ppm (m, 6H), 1,99-1,86 ppm (m, 2H), 1,76-1,65 ppm (m, 5H), 1,36-1,27 ppm (2 x tt, 6H).

Beispiel 9

Zu einer Lösung von 2,89 g 3-(o-Chlor-p-trifluormethylphenoxy)-phenol in 40 ml Aceton werden 2,76 g wasserfreies Kaliumcarbonat und 3,78 g 2-Brompropionsäure-(2-isopropylidenaminooxy-äthyl)ester gegeben, und das Gemisch wird während 6 Stunden auf Rückflusstemperatur erhitzt. Dann wird die resultierende Lösung auf 30 ml eingeengt und das Konzentrat auf 100 ml Wasser gegossen. Man extrahiert das Gemisch dreimal mit jeweils 80 ml Aethylacetat, wäscht die vereinigten organischen Phasen zweimal mit jeweils 50 ml Wasser nach, dampft das Lösungsmittel von der organischen Phase ab und erhält durch Destillation im Kugelrohrofen bei 100-170°C/0,05 mm Hg den 2-[3-(o-Chlor-p-trifluormethyl--phenoxy)-phenoxy] -propionsäure-(2-isopropylidenaminooxy--

äthyl)ester.

2,3 g des Produktes der ersten Reaktionsstufe werden in 8 ml Acetanhydrid gelöst und mit 0,6 g Kupfernitrat-trihydrat in 4 ml Acetanhydrid versetzt. Die resultierende Lösung wird 12 Stunden bei 45°C gerührt, mit weiteren 0,3 g Kupfernitrat-trihydrat versetzt und weitere 25 Stunden gerührt. Dann wird das Reaktionsgemisch auf 50 g Eis/Wasser gegossen und das wässrige Gemisch dreimal mit jeweils 50 ml Aethylacetat extrahiert. Man wäscht die vereinigten organischen Phasen neutral, engt sie ein und chromatographiert den öligen Rückstand an der hundertfachen Menge Kieselgel G mit n-Hexan/Aethylacetat (4:1). Es wird nach dem Abdampfen des Lösungsmittels der 2-[5-(o-Chlor-p-trifluormethyl--phenoxy) -2-nitrophenoxy]-propionsäure -(2-isopropylidenaminooxy-äthyl)ester in Form eines Oels isoliert, $^{1}$H-NMR ($CDCl_3$, 60 MHz) 8,05-6,40 ppm (mehrere Signale, 6H), 4,85 ppm (q, 1H), 4,31 ppm (m, 4H), 1,82 ppm (s, 3H), 1,78 ppm (s,3H), 1,72 ppm (d, 3H). Dieses Produkt ist auch chromatographisch identisch mit dem zweiten Endprodukt des Beispiels 7.

## Beispiel 10

Eine Lösung von 1,89 g 2-Hydroxypropionsäure-(2-isopropylidenaminooxy-äthyl)ester in 5 ml absolutem Dioxan wird während 15 Minuten bei 25-30°C zu 0,48 g einer 55%-igen Oel-Dispersion von Natriumhydrid in 10 ml absolutem Dioxan zugetropft, und das Gemisch wird 30 Minuten nachgerührt. Anschliessend wird eine Lösung von 3,62 g 2-Chlor-4-trifluormethyl-phenyl-3,4-dinitrophenyl-äther in 10 ml absolutem Dioxan während 10 Minuten zugetropft; dabei steigt die Temperatur auf 30°C an.

Das Reaktionsgemisch wird 1 Stunde bei Raumtemperatur nachgerührt und das Lösungsmittel im Vakuum abdestilliert.

Der Rückstand wird in Diäthyläther gelöst, mit Wasser neutral gewaschen, über wasserfreiem Natriumsulfat getrocknet und zur Trockene eingedampft. Schliesslich wird das Rohprodukt an Kieselgel mit Aethylacetat/n-Hexan (1:5) chromatographisch gereinigt. Man erhält den 2-[5-(o-Chlor-p-trifluormethylphenoxy) -2-nitrophenoxy]-propionsäure -(2-isopropylidenaminooxy-äthyl)ester, $^{1}H$-NMR ($CDCl_3$, 400 MHz) 7,93 ppm (d, 1H), 7,79 ppm (d, 1H), 7,58 ppm (q, 1H), 7,18 ppm (d, 1H), 6,59 ppm (d, 1H), 6,49 ppm (q, 1H), 4,81 ppm (q, 1H), 4,37 ppm (m, 2H), 4,15 ppm (t, 2H), 1,84 ppm (s, 3H), 1,79 ppm (s, 3H), 1,71 ppm (d, 3H).

II. Herstellung der Ausgangsmaterialien:

## Beispiel 11

Die in den Beispielen 4 und 9 als Ausgangsmaterialien verwendeten Carbaminsäureester können wie folgt hergestellt werden:

Zu einer auf 0°C gekühlten Lösung von 20 g 2-Hydroxyäthylcarbaminsäure-äthylester und 13,1 g Pyridin in 80 ml Diäthyläther wird eine Lösung von 32,4 g 2-Brompropionsäurebromid in 70 ml Diäthyläther innerhalb einer Stunde unter Eiskühlung zugetropft. Man rührt danach das Reaktionsgemisch 45 Minuten bei Raumtemperatur. Zur Aufarbeitung wird das Gemisch durch Celite abgenutscht und das Filtrat auf Wasser gegossen. Die wässrige Phase wird zweimal mit Diäthyläther extrahiert, und die vereinigten organischen Phasen werden zweimal mit Wasser und einmal mit gesättigter Kochsalzlösung

gewaschen, über wasserfreiem Natriumsulfat getrocknet und zur Trockene eingedampft. Man erhält den 2-(2-Brompropionyloxy)-äthylcarbaminsäure-äthylester als farbloses Oel, das entweder ohne weitere Reinigung zur Reaktion eingesetzt werden kann oder durch Chromatographie an Kieselgel mit n-Hexan/Diäthyläther (1:1) weiter gereinigt werden kann; $n_D^{20}$ 1,4770.

In analoger Weise erhält man beim Einsatz von:

- 2-Hydroxyäthylcarbaminsäure-methylester und 2-Brompropionsäurebromid den 2-(2-Brompropionyloxy)-äthylcarbaminsäure-methylester, $n_D^{20}$ 1,4799.

- 2-Hydroxyäthylcarbaminsäure-äthylester und Bromacetylbromid den 2-Bromacetyloxy-äthylcarbaminsäure-äthylester, $n_D^{20}$ 1,4853.

- 2-Hydroxyäthylcarbaminsäure-methylester und Bromacetylbromid den 2-Bromacetyloxy-äthylcarbaminsäure-methylester, $n_D^{20}$ 1,4891.

- 1-Aethyl-2-hydroxy-äthylcarbaminsäure-äthylester und 2-Brompropionsäurebromid den 1-Aethyl-2-(2-brompropionyloxy)-äthylcarbaminsäure-äthylester, $n_D^{20}$ 1, 4736.

- 2-Hydroxyäthylcarbaminsäure-isobutylester und 2-Brompropionsäurebromid den 2-(2-Brompropionyloxy)-äthylcarbaminsäure-isobutylester, $n_D^{20}$ 1,4731.

- 2-Hydroxypropylcarbaminsäure-methylester und 2-Brompropionsäurebromid den 2-(2-Brompropionyloxy)-propylcarbaminsäure-methylester, $n_D^{20}$ 1,4764.

- N-(2-Hydroxyäthyl)-N-methyl-carbaminsäure-methylester und 2-Brompropionsäurebromid den N-[2-(2-Brompropionyloxy)-äthyl]-N-methyl-carbaminsäure-methylester, $^1H$-NMR ($CDCl_3$, 60 MHz) 4,35 ppm (q, 1H), 4,32 ppm (t, 2H), 3,75 ppm (s, 3H), 3,58 ppm (t, 2H), 3,0 ppm (s, 3H), 1,82 ppm (d, 3H).

- 2-Isopropylidenaminooxy-äthanol und 2-Brompropionsäurebromid den 2-Brompropionsäure-(2-isopropylidenaminooxy-äthyl)ester, $n_D^{20}$ 1,4710.

## Beispiel 12

Die im Beispiel 5 als Ausgangsmaterial verwendete D-2-[5-(2-Chlor-p-trifluormethyl-phenoxy)-2-nitrophenoxy]-propionsäure kann wie folgt hergestellt werden:

2,0 g 5-(o-Chlor-p-trifluormethyl-phenoxy)-2-nitrophenol und 1 g Kaliumcarbonat werden in 30 ml Acetonitril während 30 Minuten bei Rückflusstemperatur erhitzt. Anschliessend werden zum warmen Gemisch 1,8 g L-2-Tosyloxy-propionsäure-äthyläther zugetropft, und das Gemisch wird 1,5 Stunden bei Rückflusstemperatur reagieren gelassen. Zur Aufarbeitung wird das Gemisch auf Eiswasser gegossen und die wässrige Phase zweimal mit Diäthyläther extrahiert. Dann werden die vereinigten organischen Phasen mit 2N Salzsäure, Wasser und gesättigter Kochsalzlösung gewaschen, über wasserfreiem Natriumsulfat getrocknet und zur Trockene eingedampft. Der Rückstand wird an Kieselgel unter Verwendung von n-Hexan/Aethylacetat (9:1) als Laufmittel chromatographisch gereinigt. Man erhält den D-2-[5-(o-Chlor-p-trifluormethyl-phenoxy)-2-nitrophenoxy]-propionsäure-äthylester, $[\alpha]_D^{22}$ -39,26° (c = 0,98% in $CHCl_3$); $^1H$-NMR ($CDCl_3$, 60 MHz) 8,1 - 6,46 ppm (m, 6H), 4,8 ppm (q, 1H), 4,22 ppm (q, 2H), 1,70 ppm (d, 3H), 1,23 ppm (t, 3H).

Aus obigem Produkt erhält man durch Verseifung mit Kaliumhydroxid in Methanol/Wasser bei Raumtemperatur die D-2-[5-(o-Chlor-p-trifluormethyl-phenoxy)-2-nitrophenoxy]-propionsäure, $[\alpha]_D^{22}$ -19,25° (c = 1,04% in $CHCl_3$).

0138183

III. Formulierungsbeispiel:

Beispiel 13

Zur Herstellung eines emulgierbaren Konzentrates werden die nachstehend aufgeführten Bestandteile miteinander vermischt:

| | |
|---|---|
| Verbindung der Formel I | 250 g/l |
| Nonylphenol-(10)-äthoxylat | 50 g/l |
| Dodecylbenzolsulfonsäure-Calciumsalz | 25 g/l |
| Lösungsmittelgemisch aus Alkylbenzolen | auf 1000 ml |

Das so erhaltene Konzentrat emulgiert spontan in Wasser. Die gebildete Emulsion eignet sich als gebrauchsfertige Spritzbrühe.

Patentansprüche

1. Verbindungen der allgemeinen Formel

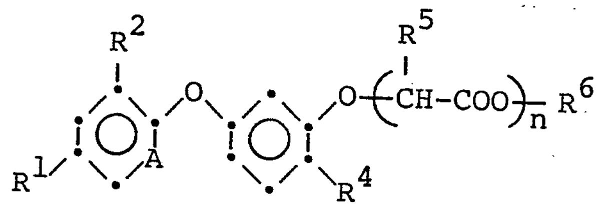

I

worin A $CR^3$ oder N,

$R^1$ Halogen oder Trifluormethyl,

$R^2$ und $R^3$ unabhängig voneinander Wasserstoff, Halogen, Nitro oder Cyano,

$R^4$ Halogen, Nitro oder Cyano,

$R^5$ Wasserstoff oder $C_{1-3}$-Alkyl,

n 0 oder 1,

$R^6$ eine der folgenden Gruppen (a) - (c)

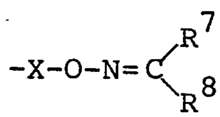

(a)

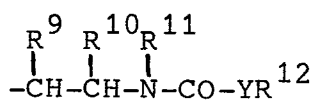

(b)

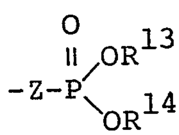

(c)

$R^7$ $C_{1-6}$-Alkyl,

$R^8$ $C_{1-6}$-Alkyl oder $C_{1-6}$-Alkoxy,

oder $R^7$ und $R^8$ zusammen mit dem Kohlenstoffatom, an das sie geknüpft sind, einen $C_{5-7}$-Cycloalkanring,

X Methylen, Aethylen oder Aethyliden,

$R^9$ und $R^{10}$ unabhängig voneinander Wasserstoff oder $C_{1-4}$-Alkyl,

$R^{11}$ Wasserstoff oder Methyl,

$R^{12}$ $C_{1-4}$-Alkyl oder 2-Chloräthyl,

Y Sauerstoff oder Schwefel,

$R^{13}$ und $R^{14}$ unabhängig voneinander $C_{1-6}$-Alkyl

und Z $C_{1-3}$-Alkylen bedeuten.

2. Verbindungen nach Anspruch 1, worin A CH bedeutet.

3. Verbindungen nach Anspruch 1 oder 2, worin $R^1$ Trifluormethyl bedeutet.

4. Verbindungen nach einem der Ansprüche 1 bis 3, worin $R^2$ Chlor bedeutet.

5. Verbindungen nach einem der Ansprüche 1 bis 4, worin $R^4$ Nitro bedeutet.

6. Verbindungen nach einem der Ansprüche 1 bis 5, worin $R^5$ Wasserstoff oder Methyl bedeutet.

7. Verbindungen nach einem der Ansprüche 1 bis 6, worin $R^7$ und $R^8$ unabhängig voneinander Methyl oder Aethyl bedeuten.

8. Verbindungen nach Anspruch 1, worin A CH, $R^1$ Trifluormethyl, $R^2$ Halogen, $R^4$ Halogen oder Nitro, $R^7$ und $R^8$ unabhängig voneinander $C_{1-6}$-Alkyl, $R^{12}$ $C_{1-4}$-Alkyl und Y Sauerstoff bedeuten.

9. 2-[5-(o-Chlor-p-trifluormethyl-phenoxy)-2-nitrophenoxy]-propionsäure-isopropylidenaminooxymethylester.

10. 2-[5-(o-Chlor-p-trifluormethyl-phenoxy)-2-nitrophenoxy]-propionsäure-(2-isopropylidenaminooxy-äthyl)ester.

11. N-{[2-[5-(o-Chlor-p-trifluormethyl-phenoxxy)-2-nitrophenoxy]-propionyloxy]-äthyl}-N-methyl-carbaminsäuremethylester.

12. Eine Verbindung nach Anspruch 1, ausgewählt aus:

2-Propanon-O-[[5-(o-chlor-p-trifluormethyl-phenoxy)-2-nitrophenoxy]methyl]oxim,

2-[5-(o-Chlor-p-trifluormethyl-phenoxy)-2-nitrophenoxy]-äthylcarbaminsäure-methylester,

2-[5-(o-Chlor-p-trifluormethyl-phenoxy)-2-nitrophenoxy]-äthylcarbaminsäure-äthylester,

2-[2-[5-(o-Chlor-p-trifluormethyl-phenoxy)-2-nitrophenoxy]-propionyloxy]-äthylcarbaminsäure-methylester,

2-[2-[5-(o-Chlor-p-trifluormethyl-phenoxy)-2-nitrophenoxy]-propionyloxy]-äthylcarbaminsäure-äthylester,

2-[[5-(o-Chlor-p-trifluormethyl-phenoxy)-2-nitrophenoxy]acetyloxy]-äthylcarbaminsäure-methylester,

2-[[5-(o-Chlor-p-trifluormethyl-phenoxy)-2-nitrophenoxy]acetyloxy]-äthylcarbaminsäure-äthylester,

2-[2-[5-(o-Chlor-p-trifluormethyl-phenoxy)-2-nitrophenoxy]-propionyloxy]-propylcarbaminsäure-methylester,

1-Aethyl-2-[2-[5-(o-chlor-p-trifluormethyl-phenoxy)-2-nitrophenoxy]-propionyloxy]-äthylcarbaminsäure-äthylester,

2-[2-[5-(o-Chlor-p-trifluormethyl-phenoxy)-2-nitrophenoxy]-propionyloxy]-äthylcarbaminsäure-isobutylester,

2-[D-2-[5-(o-Chlor-p-trifluormethyl-phenoxy)-2-nitrophenoxy]-propionyloxy]-äthylcarbaminsäure-äthylester,

2-[D-2-[5-(o-Chlor-p-trifluormethyl-phenoxy)-2-nitrophenoxy]-propionyloxy]-äthylcarbaminsäure-methylester,

2-[DL-2-[5-(o-Chlor-p-trifluormethyl-phenoxy)-2-nitrophenoxy]-propionyloxy]-äthylphosphonsäure-diäthylester,

2-[[5-(o-Chlor-p-trifluormethyl-phenoxy)-2-nitrophenoxy]acetyloxy]-äthylphosphonsäure-diäthylester,

1-[2-[5-(o-Chlor-p-trifluormethyl-phenoxy)-2-nitrophenoxy]-propionyloxy]-äthylphosphonsäure-diäthylester,

3-[5-(o-Chlor-p-trifluormethyl-phenoxy)-2-nitrophenoxy]-propylphosphonsäure-diäthylester,

[2-[5-(o-Chlor-p-trifluormethyl-phenoxy)-2-nitrophenoxy]-propionyloxy]methylphosphonsäure-diäthylester und

3-[2-[5-(o-Chlor-p-trifluormethyl-phenoxy)-2-nitrophenoxy]-propionyloxy]-propylphosphonsäure-diäthylester.

13. Eine Verbindung nach Anspruch 1, ausgewählt aus:

2-⁄2-[5-(o-Chlor-p-trifluormethyl-phenoxy)-2-chlor-phenoxy]-propionyloxy7-äthylcarbaminsäure-methylester,

2-⁄2-[5-(o-Chlor-p-trifluormethyl-phenoxy)-2-chlor-phenoxy]-propionyloxy7-äthylcarbaminsäure-äthylester und

2-[5-(o-Chlor-p-trifluormethyl-phenoxy)-2-chlor-phenoxy]-propionsäure-(2-isopropylidenaminooxy-äthyl)-ester.

14. Verbindungen nach Anspruch 1 als Wirkstoffe von Unkrautbekämpfungsmitteln.

15. Unkrautbekämpfungsmittel, dadurch gekennzeichnet, dass es eine wirksame Menge mindestens einer Verbindung der allgemeinen Formel

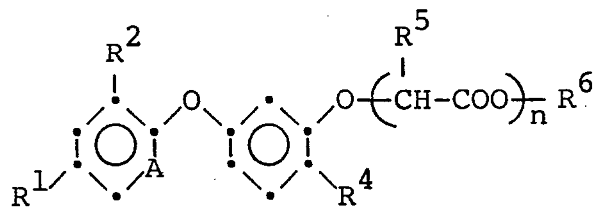

I

worin A CR$^3$ oder N,

$R^1$ Halogen oder Trifluormethyl,

$R^2$ und $R^3$ unabhängig voneinander Wasserstoff, Halogen, Nitro oder Cyano,

$R^4$ Halogen, Nitro oder Cyano,

$R^5$ Wasserstoff oder $C_{1-3}$-Alkyl,

n 0 oder 1,

$R^6$ eine der folgenden Gruppen (a) - (c)

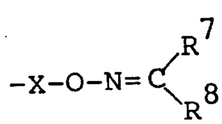

(a)

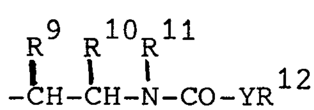

(b)

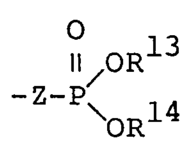

(c)

$R^7$ $C_{1-6}$-Alkyl,
$R^8$ $C_{1-6}$-Alkyl oder $C_{1-6}$-Alkoxy,
oder $R^7$ und $R^8$ zusammen mit dem Kohlenstoffatom, an das sie geknüpft sind, einen $C_{5-7}$-Cycloalkanring,
X Methylen, Aethylen oder Aethyliden,
$R^9$ und $R^{10}$ unabhängig voneinander Wasserstoff oder $C_{1-4}$-Alkyl,
$R^{11}$ Wasserstoff oder Methyl,
$R^{12}$ $C_{1-4}$-Alkyl oder 2-Chloräthyl,
Y Sauerstoff oder Schwefel,
$R^{13}$ und $R^{14}$ unabhängig voneinander $C_{1-6}$-Alkyl
und Z $C_{1-3}$-Alkylen bedeuten,
sowie Formulierungshilfsstoffe enthält.

**16. Unkrautbekämpfungsmittel nach Anspruch 15, dadurch gekennzeichnet, dass es eine wirksame Menge von 2-[5-(o-Chlor-p-trifluormethyl-phenoxy)-2-nitrophenoxy]-propionsäure-isopropylidenaminooxymethylester oder 2-[5-(o-Chlor-p-trifluormethyl-phenoxy)-2-nitro-phenoxy]-propionsäure-(2-isopropylidenaminooxy-äthyl)ester oder N-{2-[2-[5-(o-Chlor-p-trifluormethyl-phenoxy)-2-nitrophenoxy]-propionyloxy]-äthyl}-N-methylcarbaminsäure-methylester sowie Formulierungshilfsstoffe enthält.**

**17. Verfahren zur Herstellung von Verbindungen der allgemeinen Formel**

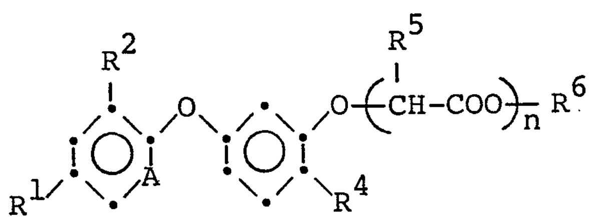

I

worin A $CR^3$ oder N,
$R^1$ Halogen oder Trifluormethyl,
$R^2$ und $R^3$ unabhängig voneinander Wasserstoff, Halogen, Nitro oder Cyano,

$R^4$ Halogen, Nitro oder Cyano,
$R^5$ Wasserstoff oder $C_{1-3}$-Alkyl,
n 0 oder 1,
$R^6$ eine der folgenden Gruppen (a) - (c)

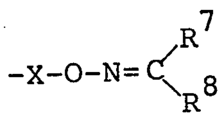 (a)

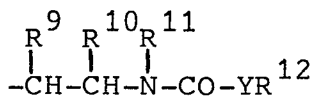 (b)

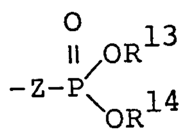 (c)

$R^7$ $C_{1-6}$-Alkyl,
$R^8$ $C_{1-6}$-Alkyl oder $C_{1-6}$-Alkoxy,
oder $R^7$ und $R^8$ zusammen mit dem Kohlenstoffatom, an das sie geknüpft sind, einen $C_{5-7}$-Cycloalkanring,
X Methylen, Aethylen oder Aethyliden,
$R^9$ und $R^{10}$ unabhängig voneinander Wasserstoff oder $C_{1-4}$-Alkyl,
$R^{11}$ Wasserstoff oder Methyl,
$R^{12}$ $C_{1-4}$-Alkyl oder 2-Chloräthyl,
Y Sauerstoff oder Schwefel,
$R^{13}$ und $R^{14}$ unabhängig voneinander $C_{1-6}$-Alkyl
und Z $C_{1-3}$-Alkylen bedeuten,

dadurch gekennzeichnet, dass man

a) ein Phenol der allgemeinen Formel

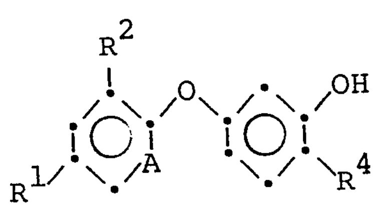 II

worin A, $R^1$, $R^2$ und $R^4$ die oben angegebenen Bedeutungen besitzen,

oder ein Alkalimetallsalz davon, mit einer Verbindung der allgemeinen Formel

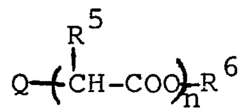

III

worin $R^5$, n und $R^6$ die oben angegebenen Bedeutungen besitzen

und Q eine Abgangsgruppe bedeutet,

umsetzt,

b) eine Säure der allgemeinen Formel

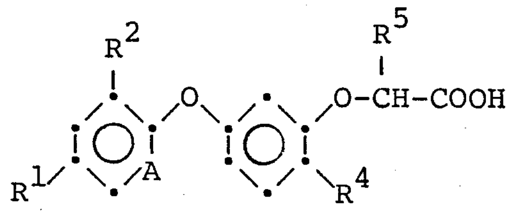

IV

worin A, $R^1$, $R^2$, $R^4$ und $R^5$ die oben angegebenen Bedeutungen besitzen,

oder ein reaktionsfähiges Derivat davon, mit einer Verbindung der allgemeinen Formel

$$U-R^6 \qquad V$$

worin $R^6$ die oben angegebene Bedeutung besitzt

und U Hydroxy oder eine Abgangsgruppe bedeutet,

umsetzt,

c) eine Verbindung der allgemeinen Formel

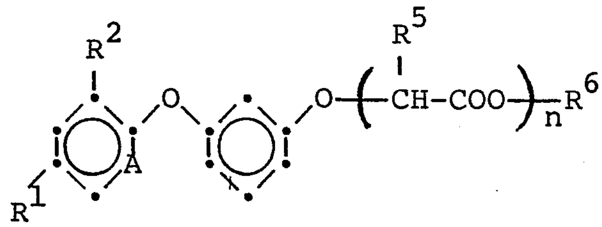

VI

worin A, $R^1$, $R^2$, $R^5$, n und $R^6$ die oben angegebenen Bedeutungen besitzen,

mit einem Metallnitrat behandelt oder

d) ein o-Dinitrobenzolderivat der allgemeinen Formel

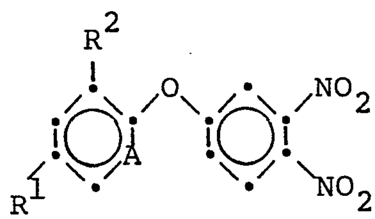

VII

worin A, $R^1$ und $R^2$ die oben angegebenen Bedeutungen besitzen,

mit einem Alkohol der allgemeinen Formel

$$HO\left(\overset{R^5}{\overset{|}{C}H}-COO\right)_n R^6 \qquad VIII$$

worin $R^5$, n und $R^6$ die oben angegebenen Bedeutungen besitzen,

oder einem Alkalimetallsalz davon umsetzt.

**18. Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 1 bis 12 bzw. eines Mittels gemäss Anspruch 15 oder 16 behandelt.**

**19. Verfahren zur Bekämpfung von Unkräutern, dadurcch gekennzeichnet, dass man das gegen Unkräuter zu schützende Gut und/oder die Unkräuter mit einer wirksamen Menge einer Verbindung gemäss Anspruch 13 behandelt.**

**20. Verwendung einer Verbindung gemäss einem der Ansprüche 1 bis 12 bzw. eines Mittels gemäss Anspruch 15 oder 16 zur Bekämpfung von Unkräutern.**

21. Verwendung einer Verbindung gemäss Anspruch 13 zur Bekämpfung von Unkräutern.

***

Patentansprüche für Oesterreich

1. Unkrautbekämpfungsmittel, dadurch gekennzeichnet, dass es eine wirksame Menge mindestens einer Verbindung der allgemeinen Formel

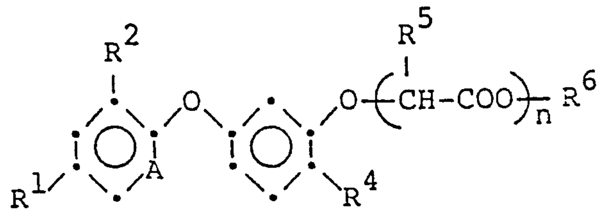

I

worin A CR$^3$ oder N,

$R^1$ Halogen oder Trifluormethyl,

$R^2$ und $R^3$ unabhängig voneinander Wasserstoff, Halogen, Nitro oder Cyano,

$R^4$ Halogen, Nitro oder Cyano,

$R^5$ Wasserstoff oder $C_{1-3}$-Alkyl,

n 0 oder 1,

$R^6$ eine der folgenden Gruppen (a) - (c)

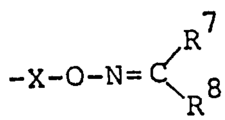

(a)

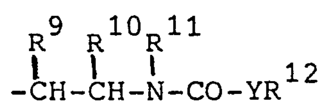

(b)

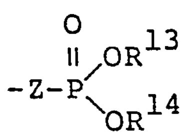

(c)

$R^7$ $C_{1-6}$-Alkyl,

$R^8$ $C_{1-6}$-Alkyl oder $C_{1-6}$-Alkoxy,

oder $R^7$ und $R^8$ zusammen mit dem Kohlenstoffatom, an das sie geknüpft sind, einen $C_{5-7}$-Cycloalkanring,

X Methylen, Aethylen oder Aethyliden,

$R^9$ und $R^{10}$ unabhängig voneinander Wasserstoff oder $C_{1-4}$-Alkyl,

$R^{11}$ Wasserstoff oder Methyl,
$R^{12}$ $C_{1-4}$-Alkyl oder 2-Chloräthyl,
Y Sauerstoff oder Schwefel,
$R^{13}$ und $R^{14}$ unabhängig voneinander $C_{1-6}$-Alkyl
und Z $C_{1-3}$-Alkylen bedeuten,

sowie Formulierungshilfsstofe enthält.

2. Unkrautbekämpfungsmittel nach Anspruch 1, worin A CH bedeutet.

3. Unkrautbekämpfungsmittel nach Anspruch 1 oder 2, worin $R^1$ Trifluormethyl bedeutet.

4. Unkrautbekämpfungsmittel nach einem der Ansprüche 1 bis 3, worin $R^2$ Chlor bedeutet.

5. Unkrautbekämpfungsmittel nach einem der Ansprüche 1 bis 4, worin $R^4$ Nitro bedeutet.

6. Unkrautbekämpfungsmittel nach einem der Ansprüche 1 bis 5, worin $R^5$ Wasserstoff oder Methyl bedeutet.

7. Unkrautbekämpfungsmittel nach einem der Ansprüche 1 bis 6, worin $R^7$ und $R^8$ unabhängig voneinander Methyl oder Aethyl bedeuten.

8. Unkrautbekämpfungsmittel nach Anspruch 1, worin A CH, $R^1$ Trifluormethyl, $R^2$ Halogen, $R^4$ Halogen oder Nitro, $R^7$ und $R^8$ unabhängig voneinander $C_{1-6}$-Alkyl, $R^{12}$ $C_{1-4}$-Alkyl und Y Sauerstoff bedeuten.

9. Unkrautbekämpfungsmittel nach Anspruch 1, dadurch gekennzeichnet, dass es eine wirksame Menge von 2-[5-(o--Chlor-p-trifluormethyl-phenoxy) -2-nitrophenoxy]-propionsäure -isopropylidenaminooxymethylester oder 2-[5-(o--Chlor-p-trifluormethyl-phenoxy) -2-nitro-phenoxy]-propionsäure -(2-isopropylidenaminooxy-äthyl)ester oder N-{2-[2--

[5-(o-Chlor-p-trifluormethyl-phenoxy)-2-nitrophenoxy]-propionyloxy7-äthyl} -N-methylcarbaminsäure-methylester sowie Formulierungshilfsstoffe enthält.

10. Unkrautbekämpfungsmittel nach Anspruch 1, dadurch gekennzeichnet, dass es eine wirksame Menge mindestens einer aus der Gruppe

2-Propanon-O-/[5-(o-chlor-p-trifluormethyl-phenoxy)-2-nitrophenoxy]methyl7oxim,
2-[5-(o-Chlor-p-trifluormethyl-phenoxy)-2-nitrophenoxy]-äthylcarbaminsäure-methylester,
2-[5-(o-Chlor-p-trifluormethyl-phenoxy)-2-nitrophenoxy]-äthylcarbaminsäure-äthylester,
2-/2-[5-(o-Chlor-p-trifluormethyl-phenoxy)-2-nitrophenoxy]-propionyloxy7-äthylcarbaminsäure-methylester,
2-/2-[5-(o-Chlor-p-trifluormethyl-phenoxy)-2-nitrophenoxy]-propionyloxy7-äthylcarbaminsäure-äthylester,
2-/[5-(o-Chlor-p-trifluormethyl-phenoxy)-2-nitrophenoxy]acetyloxy7-äthylcarbaminsäure-methylester,
2-/[5-(o-Chlor-p-trifluormethyl-phenoxy)-2-nitrophenoxy]acetyloxy7-äthylcarbaminsäure-äthylester,
2-/2-[5-(o-Chlor-p-trifluormethyl-phenoxy)-2-nitrophenoxy]-propionyloxy7-propylcarbaminsäure-methylester,
1-Aethyl-2-/2-[5-(o-chlor-p-trifluormethyl-phenoxy)-2-nitrophenoxy]-propionyloxy7-äthylcarbaminsäure-äthylester,
2-/2-[5-(o-Chlor-p-trifluormethyl-phenoxy)-2-nitrophenoxy]-propionyloxy7-äthylcarbaminsäure-isobutylester,
2-/D-2-[5-(o-Chlor-p-trifluormethyl-phenoxy)-2-nitrophenoxy]-propionyloxy7-äthylcarbaminsäure-äthylester,
2-/D-2-[5-(o-Chlor-p-trifluormethyl-phenoxy)-2-nitrophenoxy]-propionyloxy7-äthylcarbaminsäure-methylester,
2-/DL-2-[5-(o-Chlor-p-trifluormethyl-phenoxy)-2-nitrophenoxy]-propionyloxy7-äthylphosphonsäure-diäthylester,
2-/[5-(o-Chlor-p-trifluormethyl-phenoxy)-2-nitrophenoxy]acetyloxy7-äthylphosphonsäure-diäthylester,

1-/2-[5-(o-Chlor-p-trifluormethyl-phenoxy)-2-nitro-phenoxy]-propionyloxy7-äthylphosphonsäure-diäthylester,

3-[5-(o-Chlor-p-trifluormethyl-phenoxy)-2-nitro-phenoxy]-propylphosphonsäure-diäthylester,

/2-[5-(o-Chlor-p-trifluormethyl-phenoxy)-2-nitro-phenoxy]-propionyloxy7methylphosphonsäure-diäthylester und

3-/2-[5-(o-Chlor-p-trifluormethyl-phenoxy)-2-nitro-phenoxy]-propionyloxy7-propylphosphonsäure-diäthylester

ausgewählten Verbindung sowie Formulierungshilfsstoffe enthält.

11. Unkrautbekämpfungsmittel nach Anspruch 1, dadurch gekennzeichnet, dass es eine wirksame Menge mindestens einer aus der Gruppe

2-/2-[5-(o-Chlor-p-trifluormethyl-phenoxy)-2-chlor-phenoxy]-propionyloxy7-äthylcarbaminsäure-methylester,

2-/2-[5-(o-Chlor-p-trifluormethyl-phenoxy)-2-chlor-phenoxy]-propionyloxy7-äthylcarbaminsäure-äthylester und

2-[5-(o-Chlor-p-trifluormethyl-phenoxy)-2-chlor-phenoxy]-propionsäure-(2-isopropylidenaminooxy-äthyl)-ester

ausgewählten Verbindung sowie Formulierungshilfsstoffe enthält.

12. Verfahren zur Herstellung von Verbindungen der allgemeinen Formel

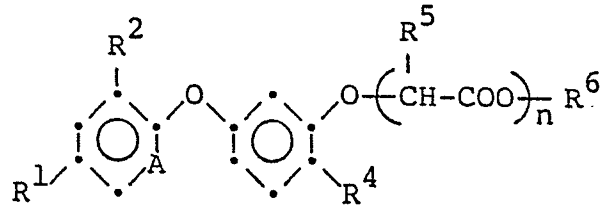

I

worin A $CR^3$ oder N,

$R^1$ Halogen oder Trifluormethyl,

$R^2$ und $R^3$ unabhängig voneinander Wasserstoff, Halogen, Nitro oder Cyano,

$R^4$ Halogen, Nitro oder Cyano,

$R^5$ Wasserstoff oder $C_{1-3}$-Alkyl,

n 0 oder 1,

$R^6$ eine der folgenden Gruppen (a) - (c)

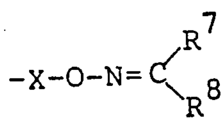

(a)

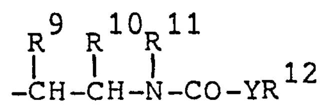

(b)

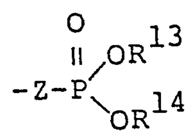

(c)

$R^7$ $C_{1-6}$-Alkyl,

$R^8$ $C_{1-6}$-Alkyl oder $C_{1-6}$-Alkoxy,

oder $R^7$ und $R^8$ zusammen mit dem Kohlenstoffatom, an das sie geknüpft sind, einen $C_{5-7}$-Cycloalkanring,

X Methylen, Aethylen oder Aethyliden,

$R^9$ und $R^{10}$ unabhängig voneinander Wasserstoff oder $C_{1-4}$-Alkyl,

$R^{11}$ Wasserstoff oder Methyl,

$R^{12}$ $C_{1-4}$-Alkyl oder 2-Chloräthyl,

Y Sauerstoff oder Schwefel,

$R^{13}$ und $R^{14}$ unabhängig voneinander $C_{1-6}$-Alkyl

und Z $C_{1-3}$-Alkylen bedeuten,

dadurch gekennzeichnet, dass man

a) ein Phenol der allgemeinen Formel

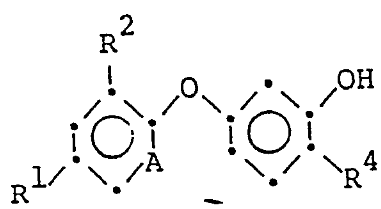

II

worin A, $R^1$, $R^2$ und $R^4$ die oben angegebenen Bedeutungen besitzen,

oder ein Alkalimetallsalz davon, mit einer Verbindung der allgemeinen Formel

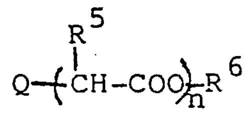

III

worin $R^5$, n und $R^6$ die oben angegebenen Bedeutungen besitzen

und Q eine Abgangsgruppe bedeutet,

umsetzt,

b) eine Säure der allgemeinen Formel

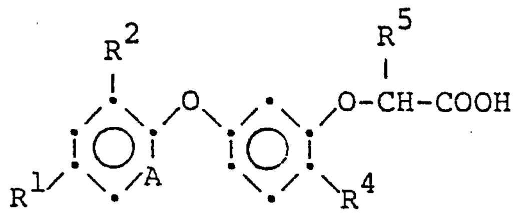

IV

worin A, $R^1$, $R^2$, $R^4$ und $R^5$ die oben angegebenen Bedeutungen besitzen,

oder ein reaktionsfähiges Derivat davon, mit einer Verbindung der allgemeinen Formel

$$U\text{-}R^6 \qquad V$$

worin $R^6$ die oben angegebene Bedeutung besitzt

und U Hydroxy oder eine Abgangsgruppe bedeutet,

umsetzt,

c) eine Verbindung der allgemeinen Formel

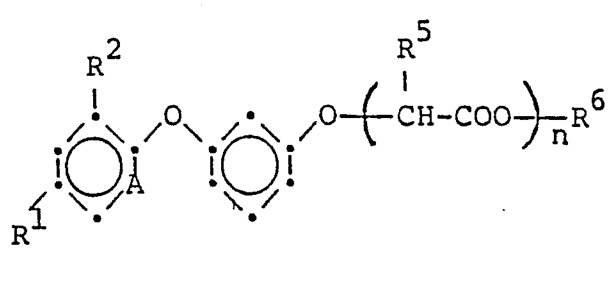

VI

worin A, $R^1$, $R^2$, $R^5$, n und $R^6$ die oben angegebenen Bedeutungen besitzen,

mit einem Metallnitrat behandelt oder

d) ein o-Dinitrobenzolderivat der allgemeinen Formel

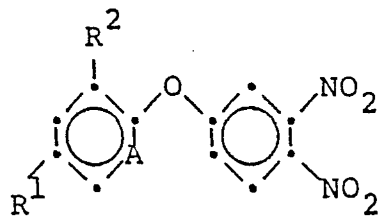

VII

worin A, $R^1$ und $R^2$ die oben angegebenen Bedeutungen besitzen,

mit einem Alkohol der allgemeinen Formel

$$HO\text{-}(\overset{R^5}{\overset{|}{C}}H\text{-}COO)_n\text{-}R^6$$

VIII

worin $R^5$, n und $R^6$ die oben angegebenen Bedeutungen besitzen,

oder einem Alkalimetallsalz davon umsetzt.

**13. Verfahren zur Herstellung eines Unkrautbekämpfungsmittels, dadurch gekennzeichnet, dass man mindestens eine der in Anspruch 1 genannten Verbindungen mit Formulierungshilfsstoffen vermischt.**

**14. Verfahren zur Bekämpfung von Unkräutern, dadurch gekennzeichnet, dass man das gegen Unkräuter zu schützende Gut und/oder die Unkräuter mit einer wirksamen Menge einer Verbindung bzw. eines Mittels gemäss einem der Ansprüche 1 bis 10 behandelt.**

**15. Verfahren zur Bekämpfung von Unkräutern, dadurch gekennzeichnet, dass man das gegen Unkräuter zu schützende Gut und/oder die Unkräuter mit einer wirksamen Menge einer Verbindung bzw. eines Mittels gemäss Anspruch 11 behandelt.**

16. Verwendung einer Verbindung bzw. eines Mittels gemäss einem der Ansprüche 1 bis 10 zur Bekämpfung von Unkräutern.

17. Verwendung einer Verbindung bzw. eines Mittels gemäss Anspruch 11 zur Bekämpfung von Unkräutern.

***